# FASCICULE DE BREVET EUROPEEN

(11) **EP 3 899 525 B1**
(45) Date de publication et mention de la délivrance du brevet: **17.04.2024**
(21) Numéro de dépôt: 19828249.3
(22) Date de dépôt: 16.12.2019
(51) Int. Cl.: G01N 30/64, G01N 30/88

(54) **MÉTHODE DE SÉPARATION ET DE QUANTIFICATION DE COMPOSÉS DANS UN MÉLANGE D'HYDROCARBURES, UTILISATION ET DISPOSITIF CORRESPONDANTS**
VERFAHREN ZUR AUFTRENNUNG UND GEHALTSBESTIMMUNG VON STOFFEN IN EINER MISCHUNG VON KOHLENWASSERSTOFFEN, ENTSPRECHENDE VERWENDUNG UND VORRICHTUNG
METHOD FOR SEPARATING AND QUANTIFYING COMPOUNDS IN A MIXTURE OF HYDROCARBONS, CORRESPONDING USE AND APPARATUS

(30) Priorité: 17.12.2018 FR 1873083
(43) Date de publication de la demande: 27.10.2021
(73) Titulaire: TotalEnergies OneTech, 92400 Courbevoie (FR); Centre National de la Recherche Scientifique, 75016 Paris (FR); Ecole Supérieure de Physique et de Chimie Industrielles de la Ville de Paris, 75005 Paris (FR)
(72) Inventeur: BAI, Yang, Beijing, Chao Yang District (CN); MOREL, Stéphanie, 38370 Saint Clair du Rhône (FR); ESSAID, Donia, 80000 Amiens (FR); THIEBAUT, Didier, 75231 Paris Cédex 5 (FR)
(74) Mandataire: Lavoix
(86) Numéro de dépôt international: PCT/EP2019/085329
(87) Numéro de publication internationale: WO 2020/127032

(56) Documents cités:
- EP-A1- 1 471 351
- WO-A1-2008/127617
- WO-A1-2015/112165
- US-A1- 2014 157 871
- RADKA MIKELOVA ET AL: "Enzymatic Reaction Coupled with Flow-Injection Analysis with Charged Aerosol, Coulometric, or Amperometric Detection for Estimation of Contamination of the Environment by Pesticides", CHROMATOGRAPHIA; AN INTERNATIONAL JOURNAL FOR RAPID COMMUNICATION IN CHROMATOGRAPHY, ELECTROPHORESIS AND ASSOCIATED TECHNIQUES, VIEWEG VERLAG, WI, vol. 67, no. 1, 2 avril 2008 (2008-04-02), pages 47-53, XP019596926, ISSN: 1612-1112

## Description

La présente invention concerne une méthode de séparation et de quantification de composés, notamment additifs polymériques et non polymériques, dans un mélange d'hydrocarbures.

Les carburants sont des mélanges d'hydrocarbures issus du raffinage de pétrole brut. Pour l'utilisation de ces mélanges d'hydrocarbures, notamment pour les utilisations automobile sous forme de carburant (essence ou gazole), des molécules de synthèse, appelées additifs, sont ajoutées en faibles proportions. Ces additifs permettent par exemple d'améliorer les performances des carburants, de passer des tests de base, etc. Ces additifs peuvent être des additifs polymériques ou non polymériques.

Il y a un intérêt à pouvoir analyser les mélanges d'hydrocarbures pour déterminer notamment la nature et la quantité des additifs, notamment additifs polymériques ou non polymériques, mis en oeuvre.

Cependant, ces mélanges d'hydrocarbures sont des mélanges complexes et il est délicat de pouvoir extraire de façon sélective des composés particuliers ou classes de composés, de pouvoir les identifier et les quantifier de manière simple et efficace.

Il y a donc un intérêt à fournir une méthode de séparation de composés, notamment additifs polymériques ou non polymériques, dans un mélange d'hydrocarbures, notamment essence ou gazole.

Le document WO 2015/112165 A1 présente une méthode d'analyse de résidus d'hydrocarbures issus du raffinage du pétrole brut comprenant la séparation par chromatographie en phase supercritique.

Un objectif de la présente invention est donc de fournir une méthode de séparation et de quantification de composés, notamment additifs, par exemple additifs polymériques ou non polymériques, dans un mélange d'hydrocarbures.

Un autre objectif de la présente invention est également de fournir une telle méthode permettant la séparation, la quantification et l'identification, de composés polymériques, notamment d'additifs polymériques, dans un mélange d'hydrocarbures.

D'autres objectifs encore apparaîtront à la lecture de la description de l'invention qui suit.

Ces objectifs sont remplis par la présente demande qui concerne une méthode de séparation et de quantification de composés contenus dans un mélange d'hydrocarbures comprenant les étapes suivantes :
a) séparation des composés contenus dans le mélange d'hydrocarbures par chromatographie en phase supercritique ;
b) quantification de chacun des composés par un détecteur d'aérosols chargés (ou CAD pour Charged Aerosol Detector en anglais).

Ainsi, la présente invention concerne l'utilisation d'un couplage chromatographie en phase supercritique et détecteur CAD pour la séparation et la quantification de composés dans un mélange d'hydrocarbures.

Dans le cadre de la présente invention, on entend par mélange d'hydrocarbures les mélanges issus du raffinage du pétrole brut. La composition chimique de ces mélanges varie notamment en fonction de la provenance géographique mais ils comprennent tous des composés aromatiques de la famille du benzène, des alcènes, des alcanes et des cycloalcanes. Comme exemple particulier des mélanges d'hydrocarbures on peut notamment citer :
- les compositions d'essence qui comprennent des hydrocarbures légers avec majoritairement de l'heptane et éventuellement des composés oxygénés organiques dans une quantité de préférence inférieure ou égale à 3,7% en volume ;
- les compositions de gazole comprenant des hydrocarbures plus lourds essentiellement cétane et éventuellement des esters méthyliques d'acides gras dans une proportion maximale de 8% en volume.

Ces mélanges d'hydrocarbures, peuvent comprendre, différents composés notamment appelés additifs, de tout type permettant notamment d'améliorer les propriétés des mélanges d'hydrocarbures telles que par exemple l'amélioration des performances, notamment le maintien de la propreté du moteur, notamment en limitant ou évitant la formation des dépôts ou en réduisant les dépôts déjà présents dans les parties internes du moteur à combustion. Ces composés peuvent être des composés polymériques ou non polymériques. On peut notamment citer les détergents, les huiles porteuses, les booster de teneur à froid, les modificateurs de frottement, des anti-odeurs, etc.

Ainsi, et de préférence, la méthode selon l'invention permet de séparer et de quantifier tous les éléments d'un mélange d'hydrocarbure autre que les hydrocarbures en tant que tels.

La méthode selon l'invention est particulièrement adaptée pour la séparation et la quantification de polymères présentant de préférence une masse molaire comprise entre 500 et 4000 g/mol, de préférence entre 1000 et 2000 g/mol. De manière particulièrement préférée, les polymères cibles selon l'invention sont des polymères comprenant une tête hydrophile polaire, de préférence de petite taille, par exemple comprise entre 15 et 150 g/mol, de préférence entre 20 et 60 g/mol, et une chaîne lipophile hydrocarbonée non polaire. Le polymère cible peut également comprendre des cycles par exemple pyrrolidone. La tête hydrophile polaire est de préférence une fonction amine (NH₂ ou amine quaternaire) ou OH (par exemple ester de sorbitan, oléate, glycérol, acide etc). De préférence, la méthode selon l'invention est particulièrement bien adaptée pour les polymères cibles présentant une fonction amine, notamment NH₂, tel que par exemple un groupe ((CH₂)₂NH)ₓ-(CH₂)_{y}-NH₂ dans lequel x est un entier compris entre 1 et 20 de préférence entre 2 et 10 et y est un entier compris entre 1 et 8, de préférence entre 2 et 5, ou un groupe CH₂-CH(CH₂CH₃)-NH₂ ou OH (par exemple ester de sorbitan, oléate, glycérol, acide etc).

De préférence, les mélanges d'hydrocarbures sont des compositions de Gazole, notamment gazole B7.

De préférence, les composés sont des polymères différents des polyéthylène glycols (PEG) et leurs dérivés.

De préférence, la phase mobile de la chromatographie en phase supercritique est un fluide supercritique comprenant du CO₂. De préférence, la phase mobile peut également comprendre un co-solvant par exemple alcool notamment isopropanol ou méthanol, de préférence méthanol. Par exemple, la phase mobile comprend 60 à 100% en volume de CO2 supercritique et 0 à 40% en volume d'alcool, par exemple isopropanol ou méthanol, de préférence méthanol. Durant l'élution, il est possible de mettre en oeuvre un gradient de phase mobile comprenant de 2 à 25% en volume d'alcool par exemple isopropanol ou méthanol, de préférence méthanol et de 98 à 75 % volume de CO₂ supercritique.

Dans le cadre de la présente invention, on entend par CO₂ supercritique un fluide de CO₂ maintenu à une température et une pression supérieures aux températures et pression critiques respectivement de 31,1°C et 7,39.10⁶ Pa.

La phase fixe (colonne de chromatographie) mise en oeuvre peut être toute colonne connue de l'homme du métier. Par exemple, on peut citer les colonnes Torus 2PIC^{©} et Torus DEA^{©} commercialisées par la société WATERS.

De préférence, le volume de mélange d'hydrocarbures injecté dans la chromatographie en phase supercritique est compris entre 2 et 10 µl, de préférence entre 5 et 8 µl.

De préférence, le débit de la phase mobile est compris entre 0,5 et 1 ml/min, de préférence entre 0,7 et 0,9ml/min.

De préférence, la colonne SFC est à une température d'environ 38°C, la pression est contrôlée pour être d'environ 1,24.10⁷ Pa.

Les composés à analyser commencent généralement à éluer de la colonne SFC à partir d'un gradient de phase mobile comprenant 17% d'alcool, de préférence MeOH, et d'une pression d'environ 3,10.10⁷ Pa (pression du système SFC).

L'étape b) est mise en oeuvre avec un détecteur CAD, de préférence CAD Corona^{©}.

L'étape b) met de préférence en oeuvre un solvant isocratique. Dans le cadre de la présente invention, on entend par solvant isocratique, un solvant dont la composition n'évolue pas au cours du temps, à la différence de l'utilisation d'un gradient de solvant. De préférence, le solvant mis en oeuvre est un solvant ou mélange de solvant polaire, par exemple alcool ou acétonitrile, par exemple méthanol ou acétonitrile. De préférence, le solvant isocratique peut comprendre 5 à 15 mM de formiate d'ammonium. Le solvant isocratique est de préférence mélangé au flux sortant de la SFC avant l'entrée dans le CAD. De préférence le débit du solvant isocratique est compris entre 0,1 et 1 ml/min, de préférence entre 0,3 et 0,6 mg/min.

De manière particulièrement avantageuse, la mise en oeuvre d'un solvant isocratique avant le détecteur CAD permet d'avoir une méthode de quantification applicable à tout composé et ce à partir d'une unique courbe étalon. En effet, de par l'utilisation du solvant isocratique selon l'invention les aires sous les pics sont directement proportionnels à la concentration en composés dans le mélange d'hydrocarbures de départ et ce quelque soit la nature du composé analysé. Ainsi, il est nécessaire de faire une courbe d'étalonnage avec au moins deux solutions de concentration connue en un composé et relier l'aire sous le pic à la concentration du composé, cette courbe d'étalonnage pourra ensuite être utilisée quelque soit le composé à analyser. L'utilisation d'une phase mobile selon l'invention, avec un gradient relativement faible, permet avantageusement également d'obtenir ce résultat. De façon avantageuse, l'utilisation d'un solvant isocratique selon l'invention permet également de s'affranchir des problèmes de ligne de base.

De préférence, en sortie de l'étape a) le milieu est nébulisé, par exemple sous un flux d'azote, puis évaporé pour éliminer la phase mobile et les composés volatils, notamment à une température comprise entre 30 et 100°C, par exemple entre 50 et 90°C. Après nébulisation et évaporation, les particules obtenues sont chargées positivement avec un flux de préférence d'azote soumis à un potentiel élevé (décharge Corona). Un électromètre mesure les particules chargées résultantes, le signal obtenu étant fonction de la concentration en analyte.

De préférence, l'étape b) est mise en oeuvre à une température comprise entre 30 et 100°C, de préférence entre 50 et 90°C.

De préférence, la pression du gaz lors de la nébulisation est comprise entre 0,2 MPa et 0,4 MPa.

De façon particulièrement avantageuse le couplage de la chromatographie fluide supercritique et du détecteur CAD permet de diminuer considérablement, les limites de détection des composés dans les mélanges d'hydrocarbures, permettant ainsi de détecter et quantifier des composés présents dans des concentrations allant jusqu'à 100 ppm voir même jusqu'à 50 ppm.

De façon avantageuse, la méthode de l'invention peut également comprendre une étape de détermination de la nature du composé à analyser (méthode d'identification) par exemple par spectrométrie de masse. Dans ce cas, le milieu issu de la colonne de séparation par fluide supercritique est mélangé au solvant isocratique puis injecté dans un séparateur. Une fraction du milieu est ensuite injectée dans le spectromètre de masse, l'autre étant injectée au niveau du détecteur CAD. Cela permet avantageusement, d'avoir en une étape la détermination de la nature des composés et leur concentration.

La présente invention concerne également un dispositif pour la mise en oeuvre de la méthode selon l'invention, ledit dispositif comprenant :
- un système de chromatographie à fluide supercritique ;
- un détecteur CAD ;
- un container comprenant un fluide supercritique ;
- optionnellement un container comprenant le co-solvant du fluide supercritique et un moyen de mélange du fluide supercritique et du co-solvant ;
- un moyen d'injection du mélange d'hydrocarbures dans le système de chromatographie à fluide supercritique ;
- un container comprenant le solvant isocratique ;
- un moyen de mélange du milieu issu du système de chromatographie à fluide supercritique et du solvant isocratique ;
- optionnellement, un séparateur ;
- optionnellement, un moyen d'injection du mélange du milieu issu du système de chromatographie à fluide supercritique et du solvant isocratique dans le séparateur ;
- optionnellement, un moyen d'injection d'une fraction issue du séparateur vers le spectromètre de masse.

Le fluide supercritique, le co-solvant, le mélange d'hydrocarbures, le solvant isocratique, étant tels que définis ci-dessus.

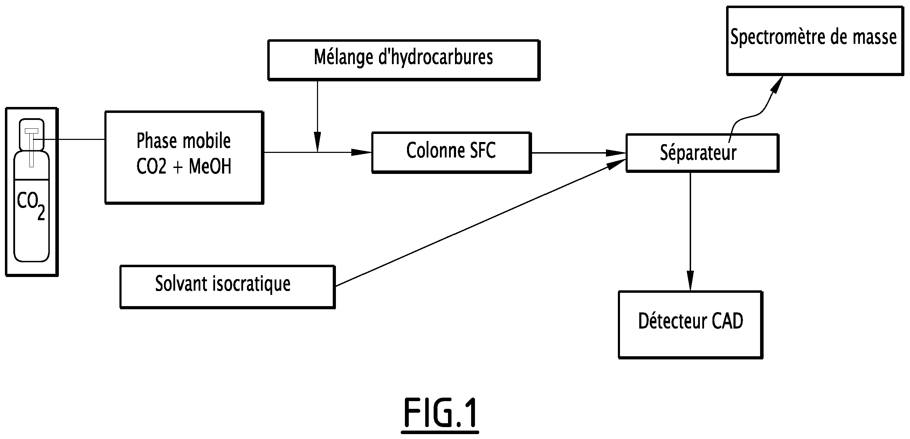

Un tel dispositif est notamment décrit à la figure 1.

[Fig 1] La figure 1 est une représentation d'un dispositif permettant la mise en oeuvre de la méthode selon l'invention.

La présente invention va maintenant être décrite à l'aide d'exemples non limitatifs.

### Exemple 1 : Séparation d'un polymère 1 dans un mélange Gazole B7

Le polymère 1 est un polymère comprenant une tête polaire de base de Mannich Les conditions de la SFC mises en oeuvre sont les suivantes:
Colonne: Torus 2Pic ^{®} commercialisée par WATERS
Volume d'injection de l'échantillon (400ppm de polymère 1 dans une composition de gazole B7) = 7µL
Débit de phase mobile : 0,90 ml/min
Gradient d'élution dans le SFC :

**[Tableau 1]**

| Temps (min) | % CO2 | % MeOH |
|---|---|---|
| 1 | 98 | 2 |
| 5 | 75 | 20 |
| 6 | 75 | 20 |

Gradient d'élution SFC
Solvant isocratique (ISM): 10mM AmF dans du MeOH avec un débit de 0,5mL/min

### Détecteur CAD :

### CORONA^{®} Veo RS de Thermofisher

L'éluent du système chromatographique est nébulisé sous un flux d'azote, l'aérosol résultant est transporté vers un tube de « séchage » où les composés volatils et la phase mobile sont évaporés. Les particules séchées seront chargées positivement avec un deuxième flux d'azote soumis à un potentiel élevé (décharge Corona). Un électromètre mesure les particules chargées résultantes. Le signal sera fonction de la concentration de l'analyte
T°C 95;
pression du gaz de nébulisation: 0,38 MPa

Les résultats de la mise en oeuvre de la méthode selon l'invention montrent que le polymère 1 élue à 5 min à 20% de méthanol. Le polymère 1 est bien séparé du gazole B7. La limite de détection est de 50 ppm et la limite de quantification est de 100 pmm.

### Exemple 2 : Séparation d'un polymère 2 dans un mélange Gazole B7

### Le polymère 2 est un polyétheramine

Les conditions d'analyse sont identiques à celle de l'exemple 1.

Les résultats de la mise en oeuvre de la méthode selon l'invention montrent que le polymère 2 élue à 4,5min à 17% MeOH. Le polymère 2 est bien séparé du gazole B7. La limite de détection est de 50 ppm et la limite de quantification est de 100 pmm.

### Exemple 3 : Séparation et quantification des polymères 1 et 2 en mélange dans un mélange Gasole B7

La méthode selon l'invention est mise en oeuvre sur plusieurs mélanges de Gazole B7 comprenant en mélange les polymères 1 et 2 à des concentrations de 0 à 1000 ppm.

Les conditions de SFC, CAD sont identiques à celles de l'exemple 1.

Chacun des polymères 1 et 2 est bien séparé, détecté individuellement et quantifié. L'aire sous le pic est équivalente pour les polymères 1 et 2 à des concentrations identiques permettant ainsi de conclure que la méthode de l'invention permet de quantifier des composés inconnus dans un mélange d'hydrocarbures à l'aide d'une seule courbe d'étalonnage.

## Revendications

1. Méthode de séparation et de quantification de composés contenus dans un mélange d'hydrocarbures issu du raffinage du pétrole brut comprenant les étapes suivantes :
a) séparation des composés contenus dans le mélange d'hydrocarbures par chromatographie en phase supercritique ;
b) quantification de chacun des composés par un détecteur d'aérosols chargés.

2. Utilisation d'un couplage chromatographie en phase supercritique et un détecteur d'aérosols chargés pour la séparation et la quantification de composés dans un mélange d'hydrocarbures issu du raffinage du pétrole brut.

3. Méthode ou utilisation selon les revendications 1 ou 2, dans laquelle les composés sont des polymères différents des polyéthylène glycols (PEG) et leurs dérivés.

4. Méthode ou utilisation selon l'une quelconque des revendications 1 à 3, dans laquelle les composés sont des composés polymériques ou non polymériques.

5. Méthode ou utilisation selon l'une quelconque des revendications 1 à 4, dans laquelle la phase mobile de la chromatographie en phase supercritique est un fluide supercritique comprenant du CO₂ et éventuellement un co-solvant par exemple alcool notamment isopropanol ou méthanol, de préférence méthanol.

6. Méthode ou utilisation selon l'une quelconque des revendications 1 à 5, dans laquelle le volume de mélange d'hydrocarbures injecté dans la chromatographie en phase supercritique est compris entre 2 et 10 µl, de préférence entre 5 et 8 µl.

7. Méthode ou utilisation selon l'une quelconque des revendications 1 à 6 dans laquelle le débit du fluide supercritique est compris entre 0,5 et 1 ml/min, de préférence entre 0,7 et 0,9 ml/min.

8. Méthode ou utilisation selon l'une quelconque des revendications 1 à 7, dans laquelle l'étape b) met en oeuvre un solvant isocratique, de préférence un solvant comprenant du méthanol ou de l'acétonitrile avec 5-15 mM de formiate d'ammonium.

9. Méthode ou utilisation selon la revendication 8, dans laquelle le débit du solvant isocratique est compris entre 0,1 et 1 ml/min, de préférence entre 0,3 et 0,6 mg/min.

10. Méthode ou utilisation selon l'une quelconque des revendications 1 à 9, dans laquelle l'étape de détection d'aérosols chargés est mise en oeuvre à une température comprise entre 30 et 100°C, de préférence entre 50 et 90°C.

11. Méthode ou utilisation selon la revendication 9, dans laquelle le milieu issu de la colonne de séparation par fluide supercritique est mélangé au solvant isocratique puis passe par un splitter, une fraction du mélange étant dirigé vers une spectromètre de masse et une autre partie vers le détecteur d'aérosols chargés.

12. Dispositif pour la mise en oeuvre de la méthode selon l'une quelconque des revendications 1 et 3 à 11, ledit dispositif comprenant :
- un système de chromatographie à fluide supercritique ;
- un détecteur d'aérosols chargés ;
- un container comprenant un fluide supercritique ;
- optionnellement un container comprenant le co-solvant du fluide supercritique et un moyen de mélange du fluide supercritique et du co-solvant ;
- un moyen d'injection du mélange d'hydrocarbures dans le système de chromatographie à fluide supercritique ;
- un container comprenant le solvant isocratique ;
- un moyen de mélange du milieu issu du système de chromatographie à fluide supercritique et du solvant isocratique ;
- optionnellement, un séparateur ;
- optionnellement, un moyen d'injection d'une fraction issue du séparateur vers le spectromètre de masse.

## Patentansprüche

1. Verfahren zur Auftrennung und Gehaltsbestimmung von Verbindungen, die in einem Kohlenwasserstoffgemisch aus der Rohölraffination enthalten sind, mit den folgenden Schritten:
a) Auftrennung der im Kohlenwasserstoffgemisch enthaltenen Verbindungen durch überkritische Phasenchromatographie;
b) Gehaltsbestimmung der einzelnen Verbindungen mit einem Detektor für geladene Aerosole.

2. Verwendung einer Kopplung aus überkritischer Phasenchromatographie und einem Detektor für geladene Aerosole zur Auftrennung und Gehaltsbestimmung von Verbindungen in einem Kohlenwasserstoffgemisch aus der Rohölraffination.

3. Verfahren oder Verwendung nach Anspruch 1 oder 2, wobei die Verbindungen Polymere sind, die sich von Polyethylenglykolen (PEG) und deren Derivaten unterscheiden.

4. Verfahren oder Verwendung nach einem der Ansprüche 1 bis 3, wobei es sich bei den Verbindungen um polymere oder nicht-polymere Verbindungen handelt.

5. Verfahren oder Verwendung nach einem der Ansprüche 1 bis 4, wobei die mobile Phase der überkritischen Phasenchromatographie ein überkritisches Fluid ist, das CO₂ und gegebenenfalls ein Co-Lösungsmittel z. B. Alkohol, insbesondere Isopropanol oder Methanol, vorzugsweise Methanol, umfasst.

6. Verfahren oder Verwendung nach einem der Ansprüche 1 bis 5, wobei das Volumen des in die überkritische Phasenchromatographie injizierten Kohlenwasserstoffgemisches zwischen 2 und 10 µl, vorzugsweise zwischen 5 und 8 µl, beträgt.

7. Verfahren oder Verwendung nach einem der Ansprüche 1 bis 6, bei dem die Durchflussrate des überkritischen Fluids zwischen 0,5 und 1 ml/min, vorzugsweise zwischen 0,7 und 0,9 ml/min, liegt.

8. Verfahren oder Verwendung nach einem der Ansprüche 1 bis 7, wobei in Schritt b) ein isokratisches Lösungsmittel eingesetzt wird, vorzugsweise ein Lösungsmittel, das Methanol oder Acetonitril mit 5-15 mM Ammoniumformiat umfasst.

9. Verfahren oder Verwendung nach Anspruch 8, wobei die Flussrate des isokratischen Lösungsmittels zwischen 0,1 und 1 ml/min, vorzugsweise zwischen 0,3 und 0,6 mg/min, liegt.

10. Verfahren oder Verwendung nach einem der Ansprüche 1 bis 9, wobei der Schritt des Nachweises von geladenen Aerosolen bei einer Temperatur zwischen 30 und 100 °C, vorzugsweise zwischen 50 und 90 °C, durchgeführt wird.

11. Verfahren oder Verwendung nach Anspruch 9, bei dem das Medium aus der Trennsäule mit überkritischem Fluid mit dem isokratischen Lösungsmittel gemischt und dann durch einen Splitter geleitet wird, wobei ein Teil der Mischung zu einem Massenspektrometer und ein anderer Teil zu einem Detektor für geladene Aerosole geleitet wird.

12. Vorrichtung zur Durchführung des Verfahrens nach einem der Ansprüche 1 und 3 bis 11, wobei die Vorrichtung umfasst:
- ein Chromatographiesystem mit überkritischem Fluid;
- einen Detektor für geladene Aerosole;
- einen Behälter, der ein überkritisches Fluid enthält;
- optional einen Behälter, der das Co-Lösungsmittel des überkritischen Fluids enthält, und ein Mittel zum Mischen des überkritischen Fluids und des Co-Lösungsmittels;
- ein Mittel zum Einspritzen des Kohlenwasserstoffgemisches in das Chromatographiesystem mit überkritischem Fluid;
- einen Behälter, der das isokratische Lösungsmittel enthält;
- ein Mittel zum Mischen des Mediums aus dem Chromatographiesystem mit überkritischem Fluid und des isokratischen Lösungsmittels;
- optional ein Trennmittel;
- optional ein Mittel zur Injektion einer Fraktion aus dem Separator in das Massenspektrometer.

## Claims

1. A method for separating and quantifying compounds contained in a hydrocarbon mixture resulting from the refining of crude oil comprising the following steps:
a) separation of the compounds contained in the hydrocarbon mixture by supercritical phase chromatography;
b) quantification of each compound using a charged aerosol detector.

2. Use of a supercritical phase chromatography coupling and a charged aerosol detector for the separation and quantification of compounds in a hydrocarbon mixture from crude oil refining.

3. A method or use according to claims 1 or 2, wherein the compounds are polymers other than polyethylene glycols (PEGs) and derivatives thereof.

4. A method or use according to any one of claims 1 to 3, wherein the compounds are polymeric or non-polymeric compounds.

5. A method or use according to any one of claims 1 to 4, in which the mobile phase of the supercritical phase chromatography is a supercritical fluid comprising CO₂ and optionally a co-solvent, for example alcohol, in particular isopropanol or methanol, preferably methanol.

6. A method or use according to any one of claims 1 to 5, wherein the volume of hydrocarbon mixture injected into the supercritical phase chromatography is between 2 and 10 µl, preferably between 5 and 8 µl.

7. A method or use according to any one of claims 1 to 6 in which the flow rate of the supercritical fluid is between 0.5 and 1 ml/min, preferably between 0.7 and 0.9 ml/min.

8. A method or use according to any one of claims 1 to 7, wherein step b) uses an isocratic solvent, preferably a solvent comprising methanol or acetonitrile with 5-15 mM ammonium formiate.

9. A method or use according to claim 8, wherein the flow rate of the isocratic solvent is between 0.1 and 1 ml/min, preferably between 0.3 and 0.6 mg/min.

10. A method or use according to any one of claims 1 to 9, in which the step of detecting charged aerosols is carried out at a temperature of between 30 and 100°C, preferably between 50 and 90°C.

11. The method or use according to claim 9, in which the medium from the supercritical fluid separation column is mixed with the isocratic solvent and then passed through a splitter, a fraction of the mixture being directed to a mass spectrometer and another part to the charged aerosol detector.

12. A device for carrying out the method according to any one of claims 1 and 3 to 11, said device comprising:
- a supercritical fluid chromatography system;
- a loaded aerosol detector;
- a container containing a supercritical fluid;
- optionally, a container containing the co-solvent for the supercritical fluid and means for mixing the supercritical fluid and the co-solvent;
- means for injecting the hydrocarbon mixture into the supercritical fluid chromatography system;
- a container containing the isocratic solvent;
- means for mixing the medium from the supercritical fluid chromatography system and the isocratic solvent;
- optionally, a separator;
- optionally, a means of injecting a fraction from the separator into the mass spectrometer.
